# EUROPEAN PATENT APPLICATION

(11) **EP 3 566 700 A1**
(43) Date of publication of application: **13.11.2019**
(21) Application number: 18382322.8
(22) Date of filing: 11.05.2018
(51) Int. Cl.: A61K 31/137, A61P 39/00, A61P 17/02, A61P 25/28, A61P 35/00, A61P 17/00, A61P 43/00, A61P 7/00

(54) **AMBROXOL FOR DNA DAMAGE REPAIR DISEASE THERAPIES**

(71) Applicant: Universitat Autònoma de Barcelona, 08193 Bellaterra (Cerdanyola del Valles) (ES); Consorcio Centro de Investigación Biomédica en Red M.P., 28029 Madrid (ES); Fundacio Institut de Recerca de l'Hospital de la Santa Creu i sant Pau, 08025 Barcelona (ES)
(72) Inventor: Surrallés Calonge, Jordi, 08025 BARCELONA (ES); Minguillón Pedreño, Jordi, 08027 BARCELONA (ES); Montanuy Escribano, Helena, 08025 BARCELONA (ES); Ramirez, Maria José, 08193 BELLATERRA (ES)
(74) Representative: ZBM Patents - Zea, Barlocci & Markvardsen

(57) **Abstract**

The present invention provides Ambroxol or a pharmaceutically salt thereof for use in the treatment and/or prevention of a disease caused by a defect in DNA damage repair mechanisms. The treatment of the invention can substantially prevent, among others, chromosome instability and cell cycle defects in cells with defective DNA damage repair mechanism. *In vivo*, the treatment of the invention improves the health and lifespan of subjects suffering from these diseases.

## Description

### Technical Field

The present invention belongs to the field of medicine. In particular, the invention provides the use of Ambroxol for the treatment of diseases caused by a defect in DNA damage repair mechanisms.

### Background Art

The human genome is constantly exposed to various sources of DNA damage and our organism commands a number of highly conserved and effective mechanisms responsible for DNA repair. If these repair mechanisms are defective due to mutations in relevant genes, diseases with DNA repair deficiencies can arise.

Today, there are a number of diseases characterized by genetic defects in DNA repair mechanisms, such as Ataxia telangiectasia, Nijmegen breakage syndrome, Werner syndrome, Seckel syndrome, Bloom syndrome, Fanconi anemia, Xeroderma pigmentosum, Cockayne syndrome, trichothiodystrophy, among others. Although heterogeneous in respect to symptoms, these rare disorders share many clinical features such as growth retardation, neurological disorders, premature ageing, skin conditions including abnormal pigmentation, telangiectasia, xerosis cutis, pathological wound healing as well as an increased risk of developing different types of cancer.

Currently, the therapeutic strategies to combat these diseases are very limited and sometimes are focused on merely avoiding the causes of DNA damage, such as the UV irradiation. Among said therapeutic strategies, the ROS scavenger N-acetylcysteine (NAC) has been proposed as a therapeutic candidate to ameliorate diseases related to defects in DNA damage repair mechanisms (such as age-relating diseases). In this regard, promising results in cellular models of Fanconi anemia have been reported (c.f. Ponte F. et al., "Improvement of genetic stability in lymphocytes from Fanconi anemia patients through the combined effect of α-lipoic acid and N-acetylcysteine" Orphanet J Rare Dis. 2012, vol. 7, pp. 28).

In spite of the efforts made, however, there is still a great need for therapies to tackle diseases caused by DNA repair defects.

### Summary of Invention

The present inventors have surprisingly found that the administration of Ambroxol is capable of rescuing the spontaneous and chemically-induced chromosome fragility and cell cycle alterations of cells with impaired DNA damage repair mechanisms, as shown in the Examples below.

It is well-known in the state of the art that defects in the DNA damage repair machinery trigger mitochondrial alterations that result in increased levels of Reactive Oxygen Species (ROS) that exacerbate chromosomal defects, which, in turn, can lead to further alterations in the cell cycle. The results provided in the Examples below show that Ambroxol is capable of reverting the negative effect of the accumulation of ROS species on chromosomes.

The defects in the DNA repair machinery can further produce organ dysfunction, cancer, and even premature aging, leading to a dramatically reduction in life expectancy.

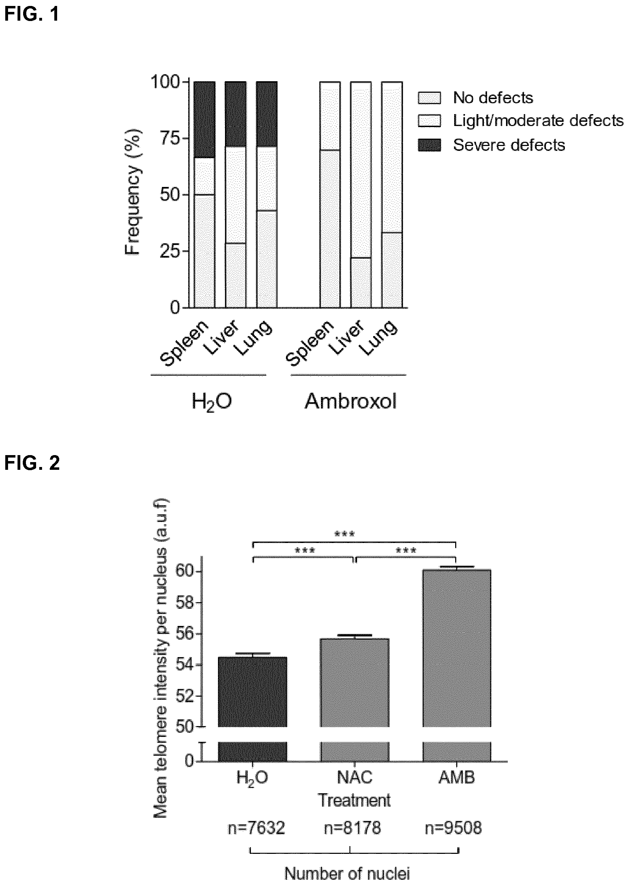

Importantly, the inventors have found that when administered *in vivo* to a murine model of a disease caused by a defect in the DNA repair machinery, Ambroxol was able to increase animal survival and health, alleviate organ dysfunction (see Fig. 1 and Table 1), and prevent premature aging (as measured by telomere length, see Fig. 2). That is, Ambroxol can substantially minimize the negative effects caused by defective DNA repair machinery by reducing the oxidative stress of cells and thereby preventing chromosome alterations, organ dysfunction and premature aging.

Consequently, Ambroxol may substantially increase the life expectancy of patients suffering this kind of diseases. As it is shown in the examples below, the administration of Ambroxol maintained a mouse model of DNA damage repair disease alive and healthy for at least one year, while 60% of the untreated mice died or were sacrificed due to poor health status during the trial.

Altogether, these data allow concluding that Ambroxol may be useful in the treatment of diseases caused by DNA damage repair defects.

Therefore, the present invention provides the Ambroxol or a pharmaceutically salt thereof for use in the treatment and/or prevention of a disease caused by a defect in DNA damage repair mechanism. This aspect can be alternative formulated as the use of Ambroxol or a pharmaceutically salt thereof for the manufacture of a medicament for the treatment and/or prevention of a disease caused by a defect in DNA damage repair mechanism. This aspect can be alternatively formulated as a method for the treatment and/or prevention of a disease caused by a defect in DNA damage repair mechanism, the method comprising administering a therapeutically effective amount of Ambroxol or a pharmaceutically salt thereof to a subject in need thereof.

Ambroxol is a well-known mucolytic drug authorized by several regulatory agencies to be used as expectorant to help make the mucus thinner and therefore easier to be cleared away in patients with short- or long-term diseases of the lungs or airways.

Unexpectedly, the present inventors have found that Ambroxol can be used as an efficient therapy in the treatment of diseases due to DNA damage repair defects, such as Fanconi anemia. As it can be seen in the Examples below, Fig. 1 and Table 1, administering Ambroxol to a Fanconi anemia's animal model improved the life-span, health, organ homeostasis and telomere length of the animals.

Importantly, the inventors have also found that the effect provided by Ambroxol was remarkably better than the results provided by N-acetylcysteine (see the Examples below). As it is shown in Fig. 2, when NAC was administered to a Fanconi anemia mice model, a modest improvement in the length of telomeres was detected when compared with control treatment (water). Surprisingly, when Ambroxol was administered, the improvement in telomere's length was remarkably higher, in particular about 5-fold higher, with respect to NAC. This is indicative that Ambroxol is substantially more potent in counteracting the premature aging symptoms characteristic of Fanconi anemia disease. And that Ambroxol can be more effective in increasing the life expectancy of the patient.

Therefore, the present invention represents a great step forward in the field of DNA repair diseases treatments.

### Brief Description of Drawings

Fig. 1 shows organ physiopathology on Fanca-deficient mice treated with Ambroxol. Sick untreated Fanca-deficient mice show more severe organ defects than Ambroxol treated mice. Graph of frequency of severe (black), light or moderate (light grey) or none (dark grey) organ defects in spleen, liver and lung tissue of untreated, Ambroxol treated mice.
Fig. 2 shows telomere length in Fanca-deficient mice untreated or treated with NAC and Ambroxol. In particular, it shows the effect of NAC or Ambroxol treatment on telomere length analyzed by Q-FISH in spleen tissue. The average intensity of the individual telomeres in each nucleus is indicated, using "arbitrary units of fluorescence" (a.u.f). The total number of nuclei analyzed in each treatment is indicated. Mice were untreated or treated with NAC or Ambroxol for one year. Mean ± SEM is shown. Means were compared using a non-parametric Mann-Whitney test. Mean ± SEM is shown. Values were compared using a Chi-Square test. * p<0.05, ** p<0.01, *** p<0.001.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As mentioned above, the present invention provides Ambroxol or a pharmaceutically salt thereof for use in the treatment and/or prevention of a disease caused by a defect in the DNA damage repair mechanism.

Ambroxol is the International Nonproprietary Name (INN) of trans-4-(2-amino-3,5-dibromobenzylamino)cyclohexanol, and has the CAS number 23828-92-4. Ambroxol has the formula (I):

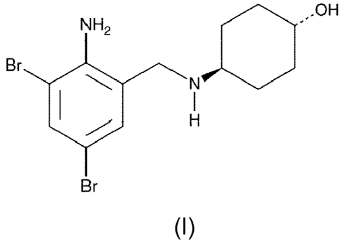

All the technical information about dose and route of administration can be obtained from AEMP's authorization.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutical acceptable salts are well known in the art. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutical acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, and ammonium. Representative alkali or alkaline earth metal salts include sodium, lithium, potassium, calcium, magnesium, and the like. Further pharmaceutical acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

The skilled person is able to prepare Ambroxol's pharmaceutically acceptable salts following routine methods.

In the present invention the expression "disease caused by a defect in DNA damage repair mechanism" refers to any condition caused by a reduced functionality of the cellular DNA repair machinery. The defect in the DNA damage repair mechanism is usually due to genetic mutations. Depending on the particular mutations, the disease can comprise at least one of the following symptoms: growth retardation, neurological disorders, premature ageing, pathological wound healing, skin condition, hematological dysfunction and cancer

In the present invention, the term "growth retardation" refers to developmental abnormalities, mental retardation and/or psychomotor retardation.

In the present invention, the term "premature aging" means that the aging process, due to mutations in DNA repair machinery, is accelerated. As a consequence of this acceleration the resulting cell/tissue has physical features which do not correspond to the actual age of the subject but to an older stage.

In the present invention, the term "skin condition" refers to a non-healthy skin state, for example, a dry skin due to rough skin, dry skin, loose and wrinkles, spots, freckles or skin diseases caused by active oxygen, and the like. In one embodiment, the skin condition is abnormal pigmentation.

In the present invention, the term "hematological dysfunction" refers to a failure in one or more of the organs forming part of the hematologic system such as bone marrow, thymus or secondary lymphatic organs (such as spleen, MALT, liver and lymph nodes). Organ(s) failure gives rise to an alteration in the levels of one or more of the cellular components forming part of the blood, such as erythrocytes, leukocytes, and platelets, coagulation factors, natural antithrombotics, and proteins of the fibrinolytic system. In one embodiment, the hematological dysfunction is due to a bone marrow failure, which gives rise to a dramatically reduced concentration of erythrocytes.

In an embodiment, the disease caused by a defect in DNA damage repair mechanism is selected from the group consisting of Ataxia telangiectasia, Nijmegen breakage syndrome, Seckel syndrome, Bloom syndrome, Cockayne syndrome, Fanconi anemia, Hutchinson Gilford progeria, Rothmund Thomson syndrome, trichothiodystrophy, Werner syndrome and Xeroderma pigmentosum. In another embodiment, the disease is Fanconi anemia. In another embodiment, the disease is Fanconi anemia caused by a mutation in the gene FANCA.

In another embodiment, optionally in combination with any of the embodiments above or below, Ambroxol or the pharmaceutically salt thereof is administered in a therapeutically effective amount in the form of a pharmaceutical composition, together with one or more pharmaceutically acceptable excipients and/or carriers.

The expression "therapeutically effective amount", as used herein, refers to the amount of Ambroxol or pharmaceutically salt thereof that, when administered, is sufficient to prevent development of, or alleviate to some extent, one or more of the symptoms of the disease which is addressed. The particular dose of agent administered according to this invention will of course be determined by the particular circumstances surrounding the case, including the route of administration, the particular condition being treated, and similar considerations.

The expression "pharmaceutically acceptable excipients and/or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The pharmaceutical composition provided by the present invention may be oral or nasal. In one embodiment, the pharmaceutical composition is for oral administration.

It is clear for the skilled person that the composition may be prepared using state of the art excipients and applying usual pharmaceutical technologies.

The dosage form may be a solid pharmaceutical composition such as tablets or coated tablets, powders, fine granules, granules, capsules e.g. hard or soft gelatin capsules, troches (pastilles), a bolus and chewable preparations containing Ambroxol or a pharmaceutically acceptable salt thereof.

Alternatively, the pharmaceutical composition may be a semisolid or liquid dosage form such as gel, e.g. a hydrogel, a cream, an ointment, a lotion, water-in-oil or oil-in-water emulsions, suspensions, aerosols, and liquid preparations such as solutions, elixirs, syrups including dry syrups.

The preparation of pharmaceutical forms of the above-mentioned kind is well-known *per* se from the prior art. The dose of Ambroxol or pharmaceutically acceptable salt containing composition of the invention to be administered may appropriately be controlled depending on the dosage forms of the desired pharmaceutical preparations.

The Ambroxol containing pharmaceutical composition of the invention may be administered to a patient in a daily dose in portions over one or several times per day if it is in the dosage form of an orally administered solid preparation such as a tablet or an orally or nasally administered liquid preparation.

The amount of the effective substance may also be formulated into a single dose, in as much as it is not unreasonable from the viewpoint of the dosage form of the pharmaceutical preparation.

In one embodiment, the solid dosage form such as a capsule, tablet, pastille, granule, a powder or a liquid or another dosage form for oral application may contain Ambroxol or a pharmaceutically acceptable salt thereof in an amount allowing to provide 15 to 250 mg, 60 to 180 mg, 30 to 150 mg, 60 to 120 mg of the active ingredient per single dose.

In the preparation of the Ambroxol containing composition, a variety of currently used additives may be employed, such as one or more of a filler, a thickening agent, a gelling agent, a binder, a disintegrator, a surfactant, a lubricant, a coating agent, a sustained release agent, a diluent and/or one or more excipients. In addition to the foregoing, the agent of the present invention may, if necessary, further comprise other additives such as a solubilizing agent, a buffering agent, a preservative, an isotonic agent, an emulsifying agent, a suspending agent, a dispersant, a hardening agent, an absorbent, an adhesive, an elasticizing agent, an adsorbent, a perfume, a coloring agent, a corrigent, an antioxidant, a humectant, a light-screening agent, a brightener, a viscosity enhancer, an oil, a tableting adjuvant, and/or an anti-static agent.

More specifically, examples of such additives include one or more excipients such as lactose, corn starch, mannitol, D-sorbitol, crystalline cellulose, erythritol and sucrose; a binder such as hydroxypropyl cellulose (HPC-L), hydroxypropyl methyl cellulose, polyvinyl pyrrolidone, methyl cellulose and gelatinized starch; a disintegrator such as calcium carboxymethyl cellulose, crosslinked sodium carboxymethyl cellulose and crosslinked polyvinyl pyrrolidone (crospovidon); a lubricant such as magnesium stearate and talc; a perfume, for instance, a flavor or an aromatic oil such as apple essence, honey flavour, 1-menthol, vanillin, lemon oil, cinnamon oil, mentha oil or peppermint oil; and/or an adsorbent such as synthetic aluminum silicate and light anhydrous silicic acid.

Moreover, it is also possible to prepare coated pharmaceutical preparations through the use of a currently used coating agent such as hydroxypropyl methyl cellulose, hydroxypropyl cellulose, methyl cellulose or polyvinyl pyrrolidone.

If necessary, a sweetener may likewise be used, such as in troches, syrups and chewable preparations among others. Specific examples of such sweeteners are mannitol, glucose, maltose, starch syrup, malt extract, maltitol, sorbitol, sucrose, unrefined sugar, fructose, lactose, honey, xylitol, hydrangea tea, saccharin, aspartame, cyclamate, Sunett®, aspartyl phenylalanine ester and other malto-oligo saccharides, and oligo saccharides such as maltosyl sucrose, isomaltyrose of reduced type and raffinose, Acesulfame potassium or any kind of sugar alcohols or mixtures thereof such as sorbitol, mannitol and/or xylitol.

As solubilisers any known solubiliser suitable in the medical sector may be used, for example polyethyleneglycols, polyoxyethylene-polyoxypropylene copolymers (e.g. poloxamer 188), glycofurol, arginine, lysine, castor oil, propyleneglycol, solketal, polysorbate, glycerol, polyvinyl pyrrolidone, lecithin, cholesterol, 12- hydroxystearic acid-PEG660-ester, propyleneglycol monostearate, polyoxy-40- hydrogenated castor oil, polyoxyl-10-oleyl-ether, polyoxyl-20-ceto-stearylether and polyoxyl-40-stearate or a mixture thereof.

Any preservatives known for use in the pharmaceutical field may be used, for example, ethanol, benzoic acid and the sodium or potassium salts thereof, sorbic acid and the sodium or potassium salts thereof, chlorobutanol, benzyl alcohol, phenylethanol, methyl-, ethyl-, propyl- or butyl-p-hydroxybenzoates, phenol, m-cresol, p-chloro-m-cresol, those selected from the group of the PHB esters, e.g. mixtures of PHB-methyl with PHB-propylesters, quaternary ammonium compounds such as benzalkonium chloride, thiomersal, phenyl-mercury salts such as nitrates, borates.

The buffer system used to achieve a desired pH value may be, for example, glycine, a mixture of glycine and HCI, a mixture of glycine and sodium hydroxide solution, and the sodium and potassium salts thereof, a mixture of potassium hydrogen phthalate and hydrochloric acid, a mixture of potassium hydrogen phthalate and sodium hydroxide solution or a mixture of glutamic acid and glutamate.

Suitable gelling agents are for example cellulose and its derivatives, like for instance methyl cellulose, carboxymethyl cellulose, hydroxypropylmethyl cellulose, poly(vinyl)alcohol, polyvinylpyrrolidones, polyacrylates, poloxamers, tragacanth, carrageenan, starch and its derivatives or any other gelling agent used in pharmaceutical technology.

Viscosity enhancers which may be mentioned are for example the aforementioned gelling agents in low quantities, glycerol, propylene glycole, polyethylene glycol or polyols, like sorbitol and other sugar alcohols.

The emulsifiers used, apart from the emulsifiers known from the prior art, may include polyoxyethylene derivatives of castor oil or polyoxyethylene alkylethers.

Suitable synthetic or natural, coloring agents known in the pharmaceutical field may be used such as Indigo carmine.

Suitable oily components which may be present are any of the oily substance known from the prior art for the preparation of pharmaceuticals, such as, for example, vegetable oils, in particular, e.g. cotton seed oil, groundnut oil, peanut oil, maize oil, rapeseed oil, sesame oil and soya oil, or triglycerides of moderate chain length, e.g. fractionated coconut oil, or isopropylmyristate, -palmitate or mineral oils or ethyloleate.

The antioxidants used may be any of the antioxidants known from the prior art, for exemple a-tocopherol, butylhydroxytoluene (BHT) or butylhydroxyanisole (BHA).

Pharmaceutical compositions containing these additives may be prepared according to any method known in this field, depending on the dosage form. It is a matter of course that further additives not explicitly discussed may be used in the formulations used according to the present invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Cell lines and Reagents.

Lymphoblastoid cell line LFA139 comes from a healthy donor, LFA55, LFA88 and LFA145 come from Fanconi anemia patients with bioallelic mutations in *FANCA* gene. These cell lines are stored in a biobank and registered in a repository in the Carlos III National Institute of Health. Lymphoblastoid cell lines were grown in RPMI medium supplemented with 20 % heat inactivated FBS, penicillin-streptomycin (100 U/ml, from biowest), sodium pyruvate (1mM, from biowest), non-essential amino acids (81,4 mg/l, from biowest) and β-mercaptoethanol (5 µM, from biowest), and maintained between 0,5-1,5x10⁶ cells per milliliter.

Ethidium Monoazide Bromide (EMA) was from ThermoFischer Scientific (Cat. N°. e1374). IGEPAL was from Sigma (Cat. N° 18896). RNAse A was from Invitrogen (Cat. N° 12091021). DAPI (Cat. N° D9542), Diepoxybutane (Cat. N° 202533-16), and acetaldehyde were from Sigma-Aldrich (Cat. N° 00071). Formaldehyde was from VWR (Cat. N° M134). Screen-well redox library was from Enzo Life Sciences (Cat. N°. BML-2835-0100). N-acetyl-cysteine (Cat. N° A9165), resveratrol (Cat. N° R5010), danazol (Cat. N° D8399), quercetin (Cat. N° Q4951), N-Prorpyl-Gallate (Cat. N° P3130), Ambroxol (Cat. N° A9797) and Apigenin (Cat. N° A3145) were from Sigma-Aldrich. D-α-Tocopherylquinone (Quimigen, Cat. N° SC-200821). Propidium iodide was from Invitrogen (Cat. N° P3566).

### In vitro flow cytometric micronuclei (FCM) assay

60,000 cells/well were plated using 96 well plates in a total volume of 120 µl/well. Cells were then treated with DEB (1,2,3,4-diepoxybutane) (0.1 µg/ml) or acetaldehyde (5 mM) with the antioxidant compound for 3 days, or for 1 hour with formaldehyde (200 µM) and the antioxidant compounds and then were washed with PBS and replaced with RPMI growth medium containing only antioxidant drugs for 3 days. After treatment cells were centrifuged at 800rpm for 8 min. Supernatant was removed using an 8-channel multichannel and the cells were resuspended by vortexing. 62.5 µl of EMA solution (0.125 mg/ml in PBS with 2 % of FBS) was added to cell suspension.

For the photo-activation step, plates were placed on ice under the visible light from a light bulb located 30 cm above the cells for 20 min. After the photo-activation step (EMA covalently binds the DNA of cells with disrupted membrane integrity-dead/dying cells), plates were protected from light exposure for the remaining steps of the staining procedure. 200 µl of cold PBS with 2% FBS were added and cells were centrifuged at 800 rpm for 8 min. Supernatant was discarded and cells were resuspended by vortexing. Cell suspension was kept in room temperature for 20 minutes before cell lysis. Cells were then lysed in a two-step procedure. 100 µl of lysis solution 1 (0.584 mg/ ml NaCl, 1 mg/ml sodium citrate, 0.3 µl/ml IGEPAL, 1 mg/ml Rnase A, 1 µg/ml DAPI) were added. Samples were incubated at room temperature for 1 h. After the incubation, 100 µl of lysis solution 2 (85.6 mg/ml sacarose, 15 mg/ml citric acid and 1 µg/ml DAPI) were added into the samples and immediately vortexed. Samples were incubated at room temperature for 30 min and were stored into a cold chamber overnight until the specimens were measured.

Flow cytometry analysis was performed with a FACS Fortessa cytometer. At least 20,000 EMA negative-DAPI positive events (divided nuclei) were gated per sample. 3 replicates were analyzed per condition. To exclude events that are not MN (micronuclei) or nucleus from live cells, different gates were used for analysis that includes forward scatter, side scatter, DAPI and EMA fluorescence. Micronuclei frequency was expressed as the number of micronuclei per thousand nuclei obtained in the G Plot. Percentage of cells arrested in G2/M phase was obtained in the H Plot.

### NAC and Ambroxol treatment in FANCA-deficient mice

Fanca-deficient mice strain was Fanca^{tm1Faw} and genetic background 129P2/OlaHsd * FVB with exon deletion 4-7 (c.f. Cheng NC et al., "Mice with a targeted disruption of the Fanconi anemia homolog Fanca", Hum Mol Genet. 2000, vol. 9(12), pp. 1805-11). 30 male mice were randomized by age (12 to 16 weeks) and weight (25 grams on average) in three experimental groups (water, NAC and AMB).

NAC (40mM) (used for comparative purposes) and Ambroxol (1mM) were administered in drinking water (filtered and UV irradiated) *ad libitum.* To monitor MN on blood cells (erythrocytes and reticulocytes) mice were tail bleeded on weeks 1, 2, 4, 6, 8, 12, 16 and 24. Hematological parameters and blood counts were also evaluated. At the end of the study mice were euthanized and bone marrow, blood and organ samples (spleen, lung, and liver) collected.

### MN-RET (micronucleated reticulocytes)

The percentage of reticulocytes and the frequency of MN in reticulocytes and erythrocytes were studied according to the protocol previously described (c.f. Balmus G et al., "A high-throughput in vivo micronucleus assay for genome instability screening in mice", Nat Protoc. 2015, vol. 10(1), pp. 205-15). Briefly, after the time of fixation with methanol, mouse blood was labeled with anti-CD71-FITC antibody (bionova, Cat. 1720-02), an effective marker of reticulocytes. At the moment of the analysis in the cytometer cells were stained with propidium iodide (2 µg/mL). A total of 200,000 erythrocytes or a minimum of 4,000 reticulocytes were captured. Analysis was performed using the FlowJo software. A total of 9 mice were studied in the NAC treated group and 7 mice in untreated or Ambroxol treated groups.

### Histopathology study

A total of 7 mice were studied in the placebo (water) group and 10 mice in the NAC or Ambroxol treated groups. Samples (spleen, liver and lung) were fixed overnight in 10 % neutral buffered formalin, embedded in paraffin, sectioned in 3 µm thick and dried. Slides were dewaxed and re-hydrated through a series of graded ethanol until water and finally stained with hematoxilin-eosin (H-E). Photographs were taken using an Olympus DP73 digital camera and a Vanox AHBS3 Olympus microscope.

### Telomere fluorescence analysis on tissue sections by Q-FISH

Quantitative telomere fluorescence in situ hybridization (Q-FISH) was performed directly on spleen tissue sections as previously described (c.f. Flores I et al., "The longest telomeres: a general signature of adult stem cell compartments", Genes Dev. 2008; vol. 22(5), pp. 654-67). Spleen tissue was hybridized with the PNA telomeric oligonucleotide (CCCTTA)3 labeled with Cy3 (PE Biosystems). After that, digital images were acquired.

Confocal microscopy was performed at room temperature with a laser-scanning microscope (TSC SP5) using a Plan Apo 63Å-1.40 NA oil immersion objective (HCX). Maximal projection of z-stack images generated using advanced fluorescence software (LAS) was analyzed with Definiens XD software package. DAPI images were used to detect telomeric signals inside each nucleus. To study telomere length, average intensity of individual telomeres of around 940 nuclei per mouse was analyzed. To study short telomeres, intensity of around 31,000 individual telomeres was studied per mouse. A total of 7 mice were studied in the control group and 10 mice in NAC or Ambroxol groups.

### Flow-FISH

Spleenocytes were obtained from spleen crushing and then processed in suspension to perform Flow-FISH. Flow-FISH was performed as previously described with some modifications (c.f. Aubert G. "Telomere length measurement-caveats and a critical assessment of the available technologies and tools", Mutat Res.; vol. 730(1-2), pp. 59-67). Briefly, at least two independent samples of spleenocytes were processed per mouse. One of them was hybridized with a PNA telomeric oligonucleotide (CCCTTA)3 labeled with FITC probe (Applied Biosystems) and the other one without probe. After hybridization two washes were done to remove probe not properly hybridized. Then, a solution containing PBS/BSA/RNAs was added to samples and were acquired on a FACSCalibur flow cytometer. After calibration of FITC fluorescence, samples were stained with PI (20 µg/mL). Then 10,000 cells in G0/G1 were analyzed for telomere length study. Finally, all data was analyzed with FlowJo software. A total of 5 mice were studied in the untreated group and 4 mice in NAC or Ambroxol treated groups.

### Results

### Ambroxol reduces chromosome fragility and cell cycle defects in FANCA-deficient cells.

Ambroxol was analyzed with a FCM assay in 3 independent FANCA-deficient lymphoblastoid cell lines.

These cell lines, analyzed with the FCM assay, had basal chromosome fragility (around 20 micronucleus -MN- per 1000 nuclei, on average) that was enhanced by DNA damage agents DEB, acetaldehyde and formaldehyde (from 30 to 70 MN per 1000 nuclei, depending on cell line and agent). Ambroxol lowered chromosome fragility induced by these three agents in the three FANCA-deficient lymphoblastoid cell lines (from 20 to 30 MN per 1000 nuclei, depending on cell line and agent), being agents acetaldehyde and formaldehyde the ones were Ambroxol had a bigger counteracting effect.

Analyzing cell cycle as well with the FCM assay, the three FANCA-deficient cell lines had around 20% of cells in G2/M phase of the cell cycle in basal conditions. Ambroxol lowered this percentage in the three cell lines. DNA damage agents DEB, acetaldehyde and formaldehyde arrested cells on G2/M, increasing their G2/M percentages (from 40% to 70%, depending on cell line and agent). Ambroxol lowered this G2/M arrest induced by these three agents in the three FANCA-deficient lymphoblastoid cell lines (from 25% to 45%, depending on cell line and agent).

To disregard a direct effect on FA/BRCA pathway, FANCD2 ubiquitination was analyzed by Western blotting in two different FANCA-deficient lymphoblastoid cell lines. From the obtained data it could be concluded that Ambroxol was unable to restore FANCD2 ubiquitination, suggesting that the effect of Ambroxol on reducing chromosomal fragility was independent of the FA/BRCA pathway.

### FANCA-deficient mice live longer with Ambroxol chronic treatment

FANCA-deficient control mice (i.e., without treatment) started to die or to be sacrificed due to poor health status after only 3 months of treatment. After 1 year of treatment, only 40 % of control mice remained alive. This outcome was not unexpected, as in the working plan there were repeated bleedings from the starting point, and it has been described before that FANCA-deficient mice suffer from hematopoietic stem cell collapse after infection or chronic blood loss. Interestingly, postmortem pathological analysis showed that two sick mice from placebo group suffered from myeloid-shape infiltration in spleen, lung and liver, consistent with potential lymphoma tumors on these organs (Table 1).

**Table 1**

| **Spleen** | H₂O | AMB |
|---|---|---|
| • **Severe** | | |
| Spleen hyperplasia (likely linfoma) | 2/7 | 0/10 |
| • **Light/moderate** | | |
| Lymphoid hyperplasia | 1/7 | 3/10 |

| **Liver** | | |
|---|---|---|
| • **Severe** | | |
| Multiple myeloid precursor infiltration | 1/7 | 0/10 |
| Vesicular steatosis | 2/7 | 0/10 |
| • **Light/moderate** | | |
| Chronic hepatitis (perivascular) | 3/7 | 7/10 |
| Steatosis | 0/7 | 2/10 |
| Vascular congestion | 1/7 | 1/10 |

| **Lung** | | |
|---|---|---|
| • **Severe** | | |
| Myeloid-shape cellullar embolism | 1/7 | 0/10 |
| Osseous metaplasia | 1/7 | 0/10 |
| General vascular congestion | 1/7 | 0/10 |
| • **Light/moderate** | | |
| Interstitial pneumonia | 1/7 | 5/10 |
| Fibrosis | 1/7 | 0/10 |
| Pleuritis | 0/7 | 1/10 |
| BALT activation | 1/7 | 1/10 |

In the NAC treated group, only 2 out of 10 mice (20%) had to be sacrificed due to weight loss, and this was done after 10 months of starting the treatment. All mice from the Ambroxol group were alive and healthy at the end of the experiment (1 year), with no severe organ defects, indicating that Ambroxol was more effective than NAC in preventing disease progression.

Histopathological analysis showed light or moderate organ defects in all three groups, such as lymphocytic hypercelularity on spleen, perivascular chronic hepatitis on liver (compatible with spread viral infection in the animal facility, non-attributed to treatment or FA disease) or intersticial pneumonia on lung (Fig. 1 and Table 1).

### FANCA-deficient mice treated with Ambroxol have longer telomeres

Blood samples before and after six months of treatment with NAC or Ambroxol or without any treatment were obtained from each mouse. These samples were processed to study percentage of reticulocytes, and MN in erythrocytes. With those biomarkers it is possible to obtain information related to the process of erythropoiesis (% reticulocytes) or information related to chronic genotoxicity (frequency of MN in erythrocytes).

It was observed an improvement in general health condition and survival of Ambroxol treated groups related to untreated mice. While general blood biochemical markers (glucose, hemoglobin, blood cellularity, etc.) were normal in all three groups, external aspect of treated mice looked healthier and younger (placebo mice looked sicker/older as evidenced by loose and white fur or lower general activity).

It was decided then to study telomere length, an aging biomarker of reference. Telomere length was analyzed in spleen tissue sections by Q-FISH. As seen in Fig. 2, a significant increase in telomere length was observed with Ambroxol treatment and that increase was remarkably higher than the one achieved with NAC.

This effect was corroborated when only short telomeres, which compromise cell division and survival, especially of the hematopoietic lineage, were analyzed. Short telomeres were significantly shorter in untreated mice when compared with Ambroxol treated mice. Moreover, the proportion of short telomeres respect the total number of telomeres was significantly bigger in untreated mice, indicating an increase in the number of telomeres with short length.

These results suggest that Ambroxol has a protective and preventing role in Fanconi anemia disease.

### Citation List

Ponte F. et al., "Improvement of genetic stability in lymphocytes from Fanconi anemia patients through the combined effect of α-lipoic acid and N-acetylcysteine", Orphanet J Rare Dis. 2012, vol. 7, pp. 28.

Qing-Shuo Zhang et al., "Evaluation of Resveratrol and N-acetylcysteine for Cancer Chemoprevention in a Fanconi Anemia Murine Model", Pediatr. Blood Cancer 2014, vol. 61(4), pp. 740-742.

Joenje H. et al., "Oxygen-dependence of chromosomal aberrations in Fanconi's anemia", Nature 1981, vol. 290(5802), pp. 142-3.

Cheng NC et al., "Mice with a targeted disruption of the Fanconi anemia homolog Fanca", Hum Mol Genet. 2000, vol. 9(12), pp. 1805-11.

Balmus G et al., "A high-throughput in vivo micronucleus assay for genome instability screening in mice", Nat Protoc. 2015, vol. 10(1), pp. 205-15.

Flores I et al., "The longest telomeres: a general signature of adult stem cell compartments", Genes Dev. 2008; vol. 22(5), pp. 654-67.

Aubert G. "Telomere length measurement-caveats and a critical assessment of the available technologies and tools", Mutat Res.; vol. 730(1-2), pp. 59-67.

## Claims

1. Ambroxol or a pharmaceutically salt thereof for use in the treatment and/or prevention of a disease caused by a defect in DNA damage repair mechanism.

2. Ambroxol or a pharmaceutically salt thereof for use according to claim 1, wherein the disease comprises at least one of the following symptoms: growth retardation, neurological disorders, pathological wound healing, skin condition, hematological dysfunction, premature aging and cancer.

3. Ambroxol or a pharmaceutically salt thereof for use according to any of claims 1-2, wherein the disease is selected from the group consisting of Ataxia telangiectasia, Nijmegen breakage syndrome, Seckel syndrome, Bloom syndrome, Cockayne syndrome, Fanconi anemia, Hutchinson Gilford progeria, Rothmund Thomson syndrome, trichothiodystrophy, Werner syndrome and Xeroderma pigmentosum.

4. Ambroxol or a pharmaceutically salt thereof for use according to claim 3, wherein the disease is Fanconi anemia.

5. Ambroxol or a pharmaceutically salt thereof for use according to any of claims 1-4, which is administered in the form of a pharmaceutical composition together with one or more pharmaceutically acceptable excipients and/or carriers.

6. Ambroxol or a pharmaceutically salt thereof for use according to claim 5, which is administered in the form of an oral pharmaceutical composition.
